# EUROPEAN PATENT SPECIFICATION

(11) **EP 4 050 091 B1**
(45) Date of publication and mention of the grant of the patent: **07.01.2026**
(21) Application number: 22158885.8
(22) Date of filing: 25.02.2022
(51) Int. Cl.: C12M 1/26, B01L 3/02, B41J 2/14

(54) **LIQUID DISCHARGE APPARATUS AND LIQUID FILLING METHOD**
FLÜSSIGKEITSABGABEVORRICHTUNG UND FLÜSSIGKEITSFÜLLVERFAHREN
APPAREIL À DÉCHARGE DE LIQUIDE ET PROCÉDÉ DE REMPLISSAGE DE LIQUIDE

(30) Priority: 26.02.2021 JP 2021029816; 24.02.2022 JP 2022026997
(43) Date of publication of application: 31.08.2022
(73) Proprietor: Ricoh Company, Ltd., Tokyo 143-8555 (JP)
(72) Inventor: Midorikawa, Ruki, Tokyo, 143-8555 (JP); Akai, Takeshi, Tokyo, 143-8555 (JP); Yamaya, Yu, Kanagawa, 243-0460 (JP); Maeda, Reo, Tokyo, 143-8555 (JP); Matsumoto, Takahiko, Tokyo, 143-8555 (JP); Nonoyama, Yusuk, Tokyo, 143-8555 (JP); Sameshima, Tatsuya, Tokyo, 143-8555 (JP); Arai, Daisuke, Tokyo, 143-8555 (JP)
(74) Representative: Marks & Clerk LLP

(56) References cited:
- EP-A2- 1 057 643
- JP-A- 2020 092 692
- US-A1- 2016 176 191
- MIRSANDI H. ET AL: "Influence of wetting conditions on bubble formation from a submerged orifice", vol. 61, no. 3, 1 March 2020 (2020-03-01), DE, pages 83, XP055938901, ISSN: 0723-4864, Retrieved from the Internet <URL:https://link.springer.com/content/pdf/10.1007/s00348-020-2919-7.pdf> DOI: 10.1007/s00348-020-2919-7

## Description

### BACKGROUND

### Technical Field

Aspects of the present disclosure relate to a liquid discharge apparatus a liquid filling method.

### Description of the Related Art

There is known a liquid discharge apparatus employing an inkjet method, a dispenser method, or the like. For example, Japanese Unexamined Patent Application Publication No. 2020-092692 describes a configuration in which a liquid storage chamber is disposed on a displacement member to store a liquid and a liquid discharge head discharges the liquid such as a cell suspension.

### SUMMARY

In the liquid discharge apparatus having the configuration described in Japanese Unexamined Patent Application Publication No. 2020-092692, a bubble may be generated in the liquid storage chamber when liquid is supplied to the liquid discharge head, and there is room for improvement in stability to discharge the liquid.

The present disclosure has an object to stably discharge a liquid.

Embodiments of the present disclosure describe an improved liquid discharge apparatus as described in claim 1.

According to another embodiment of the present disclosure, there is provided a liquid filling method as described in claim 6.

As a result, according to the present disclosure, the liquid can be stably discharged.

### BRIEF DESCRIPTION OF THE SEVERAL VIEWS OF THE DRAWINGS

A more complete appreciation of the disclosure and many of the attendant advantages thereof will be readily obtained as the same becomes better understood by reference to the following detailed description when considered in connection with the accompanying drawings, wherein:
FIG. 1 is a schematic view illustrating an overall configuration of a liquid discharge apparatus according to embodiments of the present disclosure;
FIG. 2A is a schematic view illustrating a configuration of a supply unit during liquid suction according to embodiments of the present disclosure;
FIG. 2B is a schematic view illustrating a configuration of the supply unit during liquid feeding according to embodiments of the present disclosure;
FIG. 3 is a schematic view illustrating an overall configuration of a liquid discharge unit according to embodiments of the present disclosure;
FIG. 4 is a schematic view illustrating a positional relation between the liquid discharge unit and a tube of the supply unit according to embodiments of the present disclosure;
FIG. 5 is a block diagram illustrating a functional configuration of a controller according to a first embodiment of the present disclosure;
FIG. 6A is a schematic view of the supply unit that sucks liquid;
FIG. 6B is a schematic view of the supply unit that feeds the liquid;
FIG. 7A is a top view of the liquid discharge unit during a supply process by the supply unit;
FIGS. 7B and 7C are cross-sectional views of the liquid discharge unit taken along line A-A in FIG. 7A;
FIG. 8 is a graph illustrating an example of the supply process in which a first liquid is supplied at a lower supply rate than a second liquid;
FIG. 9 is a graph illustrating an example of the supply process in which the first liquid is supplied intermittently;
FIG. 10 is a schematic view of the supply unit that stirs the second liquid in the liquid discharge unit;
FIG. 11 is a flowchart illustrating an operation of the liquid discharge apparatus according to the first embodiment;
FIG. 12A is a top view of the liquid discharge unit in which a bubble remains;
FIG. 12B is a cross-sectional view of the liquid discharge unit taken along line A-A in FIG. 12A;
FIG. 13 is a schematic view of the liquid discharge unit illustrating a relation between a height of liquid and a contact angle;
FIG. 14 is a schematic view of the liquid discharge unit in which the first liquid is separated into two portions;
FIG. 15 is a block diagram illustrating a functional configuration of a controller according to a second embodiment of the present disclosure;
FIG. 16 is a schematic view of the liquid discharge unit from which the first liquid is drained;
FIG. 17 is a flowchart illustrating an operation of the liquid discharge apparatus according to the second embodiment;
FIG. 18 is a schematic view illustrating an operation of the liquid discharge unit according to the second embodiment;
FIGS. 19A to 19D are schematic views of the liquid discharge unit in which the contact angle between an upper surface of a film and water is 90 degrees or more;
FIGS. 20A to 20D are schematic views of the liquid discharge unit in which the contact angle between the upper surface of the film and water is less than 90 degrees; and
FIG. 21 is a schematic view of a hydrophilic film formed on the upper surface of the film.

The accompanying drawings are intended to depict embodiments of the present disclosure and should not be interpreted to limit the scope thereof. The accompanying drawings are not to be considered as drawn to scale unless explicitly noted. In addition, identical or similar reference numerals designate identical or similar components throughout the several views.

### DETAILED DESCRIPTION

In describing embodiments illustrated in the drawings, specific terminology is employed for the sake of clarity. However, the disclosure of this patent specification is not intended to be limited to the specific terminology so selected, and it is to be understood that each specific element includes all technical equivalents that have the same function, operate in a similar manner, and achieve a similar result.

As used herein, the singular forms "a," "an," and "the" are intended to include the plural forms as well, unless the context clearly indicates otherwise.

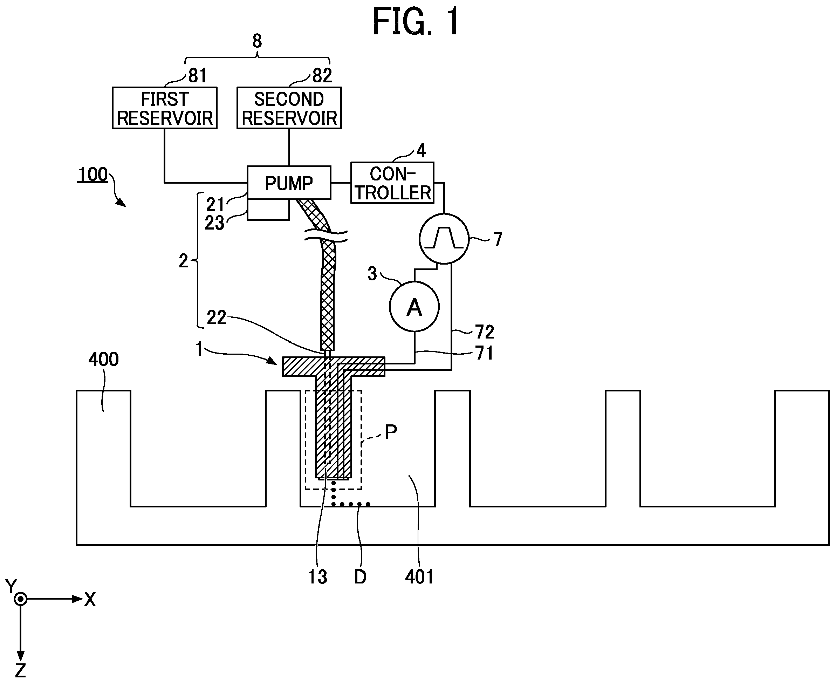

An overall configuration of the liquid discharge apparatus 100 is described with reference to FIG. 1. FIG. 1 is a schematic view illustrating the overall configuration of the liquid discharge apparatus 100 employing the inkjet method. As illustrated in FIG. 1, the liquid discharge apparatus 100 includes a liquid discharge unit 1 (also referred to as a discharge head or a print head), a supply unit 2, an ammeter 3, a controller 4, and a reservoir 8.

In the present embodiment, a direction in which liquid is discharged is defined as a Z-axis direction and a plane perpendicular to the Z-axis direction is defined as an XY plane for convenience. In the Z-axis direction, the direction of liquid discharged is a downward direction, and the opposite direction opposite to the downward direction is defined as an upward direction. An upper XY plane of each component is referred to as an upper surface, and a lower XY plane of each component is referred to as a lower surface. When the component has a cylindrical shape with a hollow body, the virtual XY plane of the component is defined as the upper surface and the lower surface.

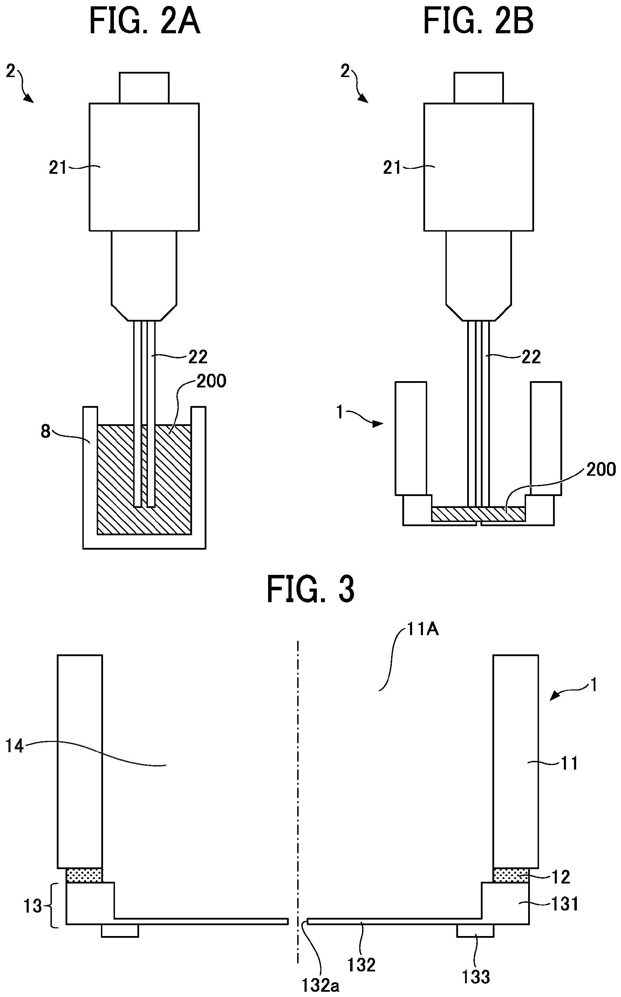

In the liquid discharge apparatus 100, the supply unit 2 supplies liquid 200 (see FIGS. 2A and 2B) stored in the reservoir 8 to the liquid discharge unit 1, the liquid discharge unit 1 discharges the liquid 200, thereby forming liquid droplets D or tissues made from a cell suspension in wells 401 which are multiple well-shaped holes formed in a well plate 400.

The reservoir 8 is provided outside the liquid discharge unit 1 and stores the liquid 200 to be supplied to the liquid discharge unit 1. The reservoir 8 includes a first reservoir 81 and a second reservoir 82. The first reservoir 81 stores a first liquid. When two types of liquids are used, the second reservoir 82 stores a second liquid different in type from the first liquid.

The supply unit 2 supplies liquid stored in the reservoir 8 to the liquid discharge unit 1. The supply unit 2 includes a pump 21, a tube 22, and an actuator 23. The supply unit 2 drives the actuator 23 to change the positions of the pump 21 and the tube 22 between a position where each of the first reservoir 81 and the second reservoir 82 is disposed and a position where the liquid discharge unit 1 is disposed to reciprocate the pump 21 and the tube 22 therebetween.

The pump 21 and the tube 22 sucks the first liquid at the position of the first reservoir 81. Then, the actuator 23 moves the pump 21 and the tube 22 to the position of the liquid discharge unit 1 and stops the pump 21 and the tube 22 at the position. Thereafter, the pump 21 and the tube 22 feed the first liquid to the liquid discharge unit 1. Note that, to suck liquid is referred to as liquid suction, and to feed liquid is referred to as liquid feeding.

Further, the pump 21 and the tube 22 sucks the second liquid at the position of the second reservoir 82. Then, the actuator 23 moves the pump 21 and the tube 22 to the position of the liquid discharge unit 1 and stops the pump 21 and the tube 22 at the position. Thereafter, the pump 21 and the tube 22 feed the second liquid to the liquid discharge unit 1. Thus, the supply unit 2 can supply each of the first liquid and the second liquid to the liquid discharge unit 1.

The liquid discharge unit 1 discharges the liquid supplied by the supply unit 2. A lower end portion of the liquid discharge unit 1 is inserted into the well 401. A micro electro mechanical system (MEMS) chip 13 is disposed at the lower end portion of the liquid discharge unit 1. As a drive waveform generation source 7 applies a drive waveform (drive voltage) to the MEMS chip 13 via wirings 71 and 72, the liquid held in the liquid discharge unit 1 is discharged as liquid droplets D into the well 401. The ammeter 3 as a detector detects a current generated in a piezoelectric element 133 (see FIG. 3) included in the MEMS chip 13 when the liquid is discharged by the MEMS chip 13.

The controller 4 controls the overall operation of the liquid discharge apparatus 100. The controller 4 causes the supply unit 2 to feed or suck liquid in response to the back electromotive force of the piezoelectric element 133 based on the current detected by the ammeter 3 to control the amount of liquid held in the liquid discharge unit 1.

Next, a configuration of the supply unit 2 is described with reference to FIGS. 2A and 2B. FIG. 2A is a schematic view illustrating a configuration of the supply unit 2 during the liquid suction, and FIG. 2B is a schematic view illustrating a configuration of the supply unit 2 during the liquid feeding. As illustrated in FIG. 2, the supply unit 2 includes the pump 21 and the tube 22. The pump 21 sucks liquid and feeds the liquid. Any pump that can suck and feed liquid may be used as the pump 21.

The tube 22 is tubular and defines a path through which the pump 21 sucks and feeds liquid. The material of the tube 22 is not particularly limited, but a flexible material including a resin material is preferable to arrange the path freely. Preferably, the tube 22 is detachably attachable to the pump 21. The tube 22 may be disposable, for example, a disposable tip, or may be cleaned to be used multiple times. The supply unit 2 may further include a supply needle below the tube 22.

As illustrated in FIG. 2A, the supply unit 2 moves the pump 21 and the tube 22 to the position of the reservoir 8 by the actuator 23 (see FIG. 1), and drives the pump 21 while inserting the distal end of the tube 22 into the reservoir 8, thereby sucking the liquid 200 stored in the reservoir 8. In addition, as illustrated in FIG. 2B, the supply unit 2 moves the pump 21 and the tube 22 to the position of the liquid discharge unit 1 by the actuator 23, and drives the pump 21 while inserting the distal end of the tube 22 into the liquid discharge unit 1, thereby feeding the liquid 200 to the inside of the liquid discharge unit 1.

Any type of actuator that can move the pump 21 and the tube 22 together is used as the actuator 23, for example, a crane that lifts and transports the pump 21 and the tube 22 together is preferable. The supply unit 2 is not limited to a configuration in which the pump 21 and the tube 22 are movable, and may have a configuration in which at least one of the pump 21 and the tube 22, the reservoir 8, and the liquid discharge unit 1 is moved by the actuator 23 or the like.

Next, a configuration of the liquid discharge unit 1 is described with reference to FIG. 3. FIG. 3 is a schematic view illustrating a configuration example of the liquid discharge unit 1, which is an enlarged view of a portion P indicated by the broken line in FIG. 1. As illustrated in FIG. 3, the liquid discharge unit 1 includes a liquid chamber member 11, an elastic member 12, and the MEMS chip 13. The MEMS chip 13 includes a holder 131, a film 132, and the piezoelectric element 133. The elastic member 12 is, for example, a glue or a double-sided adhesive tape, and the liquid chamber member 11 and the MEMS chip 13 are bonded to each other by the elastic member 12.

The liquid discharge unit 1 stores liquid in a liquid holding portion 14 defined by the liquid chamber member 11, the elastic member 12, and the holder 131. The liquid discharge unit 1 has an opening 11A to the atmosphere. The distal end of the tube 22 of the supply unit 2 is inserted into the liquid discharge unit 1 through the opening 11A. An outer shape and an inner shape of the liquid discharge unit 1 are, for example, a cylinder or a prism.

The MEMS chip 13 is manufactured by microfabricating a silicon substrate by a semiconductor process using photolithography. The MEMS chip 13 includes the holder 131, the film 132, and the piezoelectric element 133, which are integrally formed on the substrate. The substrate is not limited to silicon, and other materials such as glass may be used. The method of manufacturing the piezoelectric element 133 is not limited to the semiconductor process, and a method other than the semiconductor process, such as a process of forming a pattern with a precursor liquid of a piezoelectric material by the inkjet method, may be used. In the MEMS chip 13, the holder 131 is made of metal, silicon, ceramics, or the like.

The size of the liquid discharge unit 1 and the amount of the liquid 200 that can be stored in the liquid discharge unit 1 are not particularly limited and may be appropriately determined depending on the intended purpose. The amount of the liquid 200 is, for example, 1 µL to 1 mL. When the liquid 200 is the cell suspension in which cells are dispersed, the amount of the liquid 200 may be 1 µL to 50 µL. The controller 4 controls and changes the amount of the liquid 200 as a factor contributing to the vibration characteristics of the film 132.

The film 132 is secured to (supported by) a lower end of the holder 131. A nozzle hole 132a, which is a through hole, is disposed substantially at the center of the film 132. The liquid 200 held in the liquid discharge unit 1 is discharged as liquid droplets D from the nozzle hole 132a as the film 132 vibrates. The shape of the film 132 may be, for example, circular, elliptical, a quadrangular, or the like. The shape of the film 132 preferably corresponds to the shape of the bottom of the holder 131.

According to the invention, the film 132 is made using hydrophilic material. Although the material of the film 132 is not particularly limited, a material having a certain degree of hardness is preferable because the film 132 made of too soft material is likely to vibrate and difficult to immediately suppress the vibration when discharging is suspended. Examples of the materials of the film 132 include metal materials, ceramic materials, and polymer materials having a certain degree of hardness.

In particular, when cells are used as the sedimentary particles, the material of the film 132 is preferably a material having low adhesiveness to cells or proteins. In general, the adhesiveness to cells depends on the contact angle of a material with water. Highly hydrophilic or highly hydrophobic materials have low adhesiveness to cells. As highly hydrophilic materials, various metal materials and ceramics (metal oxides) may be used. As highly hydrophobic materials, fluororesin may be used.

Other examples of such materials include stainless steel, nickel, aluminum, and the like as the metal material, and silicon dioxide, alumina, zirconia, and the like as the ceramics. Besides these materials, the adhesiveness to cells can be reduced by coating the surface of a material. For example, the surface of the material can be coated with the metal or metal oxide materials described above or synthetic phospholipid polymers that mimic cell membrane (e.g., LIPIDURE available from NOF CORPORATION).

When the liquid 200 is absent on the film 132, the film 132 vibrates in response to a resonance period determined by the size and material thereof. When the liquid 200 is present on the film 132, the resonance period is likely to varies in response to the amount of the liquid 200 held in the liquid discharge unit 1. In general, since the inertial force increases as the amount of the liquid 200 increases, the resonance period of the film 132 is likely to increase (the resonance frequency is likely to decrease).

Preferably, the nozzle hole 132a is a substantially circular through hole disposed at the center of the film 132. In this case, the diameter of the nozzle hole 132a is not particularly limited, but are preferably twice or more the size of the sedimentary particles to prevent the nozzle hole 132a from being clogged with the sedimentary particles and to stably discharge the liquid droplets D through the nozzle hole 132a. Specifically, since the size of animal cells, particularly human cells, is generally about 5 µm to 50 µm, the diameter of the nozzle hole 132a is preferably 10 µm to 100 µm or more in accordance with the size of the cells to be used. On the other hand, when the liquid droplets D are too large, it is difficult to achieve the purpose of forming minute liquid droplets. Accordingly, the diameter of the nozzle hole 132a is preferably 200 µm or less. Therefore, in the liquid discharge apparatus 100, the diameter of the nozzle hole 132a is typically in a range of 10 µm to 200 µm.

The drive waveform generation source 7 is a signal generator that outputs a drive waveform as a drive signal to the piezoelectric element 133. The drive waveform generation source 7 outputs the drive waveform to the piezoelectric element 133, thereby deforming the film 132. As a result, the liquid 200 held in the liquid discharge unit 1 is discharged as liquid droplets D. As the film 132 is deformed in response to the drive waveform set to a predetermined cycle, the film 132 resonates and vibrates to discharge the liquid 200.

The piezoelectric element 133 is an example of a vibrator that vibrates the film 132 and is disposed on the lower surface of the film 132. The shape of the piezoelectric element 133 can be designed in accordance with the shape of the film 132. For example, when the shape of the film 132 is circular, the piezoelectric element 133 preferably has an annular shape (ring shape) around the nozzle hole 132a.

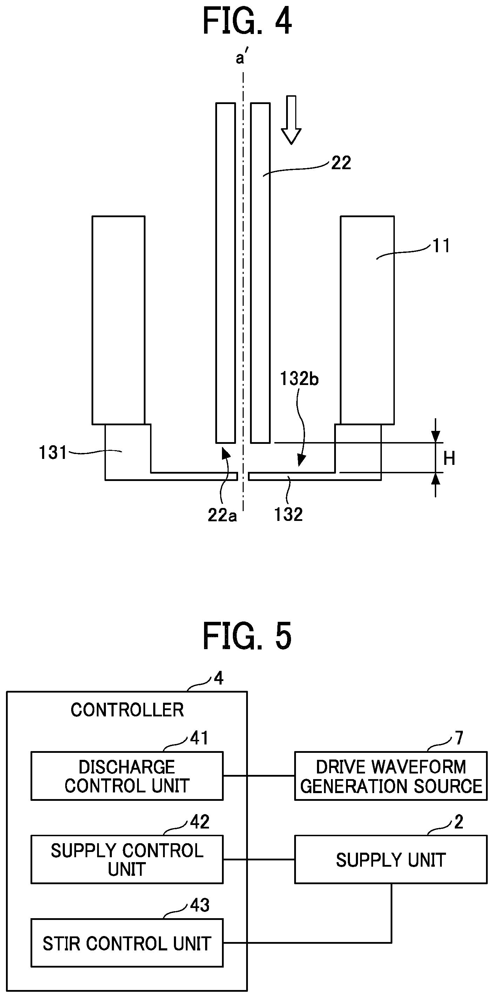

FIG. 4 is a schematic view of the liquid discharge unit 1 and the supply unit 2 illustrating an example of a positional relation between the liquid discharge unit 1 and the tube 22 of the supply unit 2 when the supply unit 2 supplies the liquid 200 to the liquid discharge unit 1.

When the supply unit 2 supplies the liquid 200 to the liquid discharge unit 1, a distance H from the lower surface 22a of the tube 22 to the upper surface 132b of the film 132 is preferably short. If the distance H is short when the liquid 200 is supplied, the kinetic energy of the liquid 200 is small when the supplied liquid 200 reaches the film 132 or the upper surface of the liquid 200 previously supplied, thereby preventing the liquid 200 from vibrating and scattering. As a result, bubbles due to vibration or scattering of the liquid 200 can be suppressed.

Preferably, the centers of the upper surface and the lower surface of the liquid chamber member 11 and the holder 131 in the liquid discharge unit 1 are on the same axis a'. Further, the lower surface 22a of the tube 22 is preferably smaller than the upper surface of the inner wall of the holder 131.

Furthermore, the tube 22 is preferably inserted into the liquid discharge unit 1 in parallel to the axis a'. As a result, when the tube 22 is inserted, the tube 22 is prevented from contacting the inner wall of the liquid discharge unit 1. Therefore, the propagation of vibration to the liquid discharge unit 1 and the generation of bubbles due to impact can be suppressed.

Here, a functional configuration of the controller 4 in the liquid discharge apparatus 100 is described with reference to FIG. 5. FIG. 5 is a block diagram illustrating an example of a functional configuration of the controller 4. As illustrated in FIG. 5, the controller 4 includes a discharge control unit 41, a supply control unit 42, and a stir control unit 43. Each of these functions is implemented by an electric circuit, and a part of these functions can be implemented by software executed by a central processing unit (CPU). These functions may be implemented by multiple circuit or multiple software.

The discharge control unit 41 causes the drive waveform generation source 7 to applies a drive waveform to the MEMS chip 13. Thus, liquid droplets D are discharged by the MEMS chip 13. The supply control unit 42 causes the supply unit 2 to supply liquid to the liquid discharge unit 1. The stir control unit 43 causes the supply unit 2 to stir the liquid supplied to the liquid discharge unit 1.

Next, the first liquid stored in the first reservoir 81 and the second liquid stored in the second reservoir 82 are described. The liquid supplied to the liquid discharge unit 1 first is referred to as the first liquid, and the liquid supplied to the liquid discharge unit 1 after the first liquid is referred to as the second liquid.

The first liquid is not particularly limited but has a contact angle with the upper surface of the MEMS chip 13 of less than 90 degrees. The contact angle indicates wettability of the first liquid with respect to the upper surface of the MEMS chip 13. The surface tension of the first liquid is preferably 30 mN/m or less, more preferably 25 mN/m or less, and still more preferably 20 mN/m or less in an environment of 25°C. Since the first liquid having a small surface tension is likely to wet and spread, the first liquid widely spreads to every corner of the entire liquid discharge unit 1. Accordingly, when the first liquid is supplied to the liquid discharge unit 1, the first liquid is spread so as not to generate a void area in the liquid discharge unit 1, thereby preventing bubbles from being generated and remaining in the liquid discharge unit 1.

When two kinds of liquids are used, the first liquid is preferably insoluble in the second liquid (that is, the first liquid and the second liquid are not compatible with each other). Accordingly, since the first liquid and the second liquid are not mixed with each other, a change in the concentration or characteristics of the second liquid is suppressed. Further, the first liquid preferably has a density lower than that of the second liquid. If the first liquid has the lower density than the second liquid and is insoluble in the second liquid, when the second liquid is supplied, the first liquid completely covers the upper surface of the second liquid like a lid, thereby preventing the second liquid from being exposed to the atmosphere. Thus, volatilization of the second liquid is suppressed, and a change in concentration of the second liquid due to drying is suppressed.

The first liquid is preferably a non-cytotoxic liquid, which means that the first liquid does not cause cell dysfunction. In this case, even when a cell suspension is used as the second liquid, the first liquid does not adversely affect cells contained in the second liquid. The first liquid preferably has a viscosity so as to be discharged by the inkjet method. Examples of the first liquid that satisfies the above-described conditions include silicone oil and liquid paraffin.

The second liquid is a liquid to be discharged, and is not particularly limited as long as the liquid discharge apparatus 100 can discharge. The second liquid may be an ink or a cell suspension. In the case of the inkjet liquid discharge apparatus 100, the second liquid preferably has a viscosity so as to be discharged by the inkjet method. In the case of a liquid discharge apparatus other than the inkjet type, the second liquid is not limited thereto.

If the contact angle of the first liquid with the upper surface of the MEMS chip 13 is less than 90 degrees, the first liquid may be a liquid to be discharged by the MEMS chip 13. In this case, the first liquid preferably has a density higher than that of the second liquid. If the first liquid has the higher density than the second liquid and is insoluble in the second liquid, when the second liquid is supplied, the second liquid completely covers the upper surface of the first liquid like a lid, thereby preventing the first liquid from being exposed to the atmosphere. Thus, volatilization of the first liquid is suppressed, and a change in concentration of the first liquid due to drying is suppressed. The second liquid is preferably silicone oil, liquid paraffin, or the like and has no cytotoxicity.

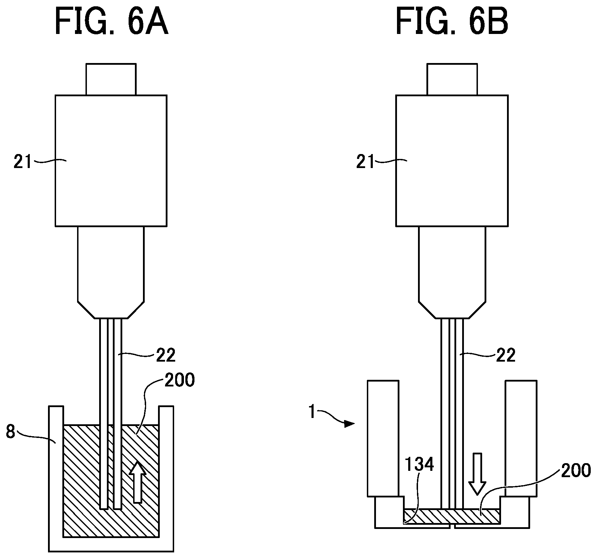

FIG. 6A is a schematic view of the supply unit 2 that sucks the liquid 200, and FIG. 6B is a schematic view of the supply unit 2 that feeds the liquid 200. As illustrated in FIG. 6A, the distal end of the tube 22 is inserted into the reservoir 8 to suck the liquid 200. Thereafter, as illustrated in FIG. 6B, the distal end of the tube 22 is inserted into the liquid discharge unit 1 to feed the liquid 200. As described above, the liquid 200 can be supplied to the liquid discharge unit 1. Both the first liquid and the second liquid can be supplied in the same manner, and the supply unit 2 sucks the first liquid from the first reservoir 81 and sucks the second liquid from the second reservoir 82.

When the distal end of the tube 22 is inserted into liquid discharge unit 1 and the liquid 200 is supplied in the liquid discharge unit 1, air may enter the liquid discharge unit 1 along with the liquid 200 supplied from the tube 22, and bubbles may be generated in the liquid discharge unit 1. Although most of the bubbles are vented from the inside of the liquid discharge unit 1, some of the bubbles may adhere to a wall of the liquid discharge unit 1 and remain in the liquid discharge unit 1. In particular, bubbles are likely to remain in a corner 134 where surfaces of the liquid discharge unit 1 intersect each other.

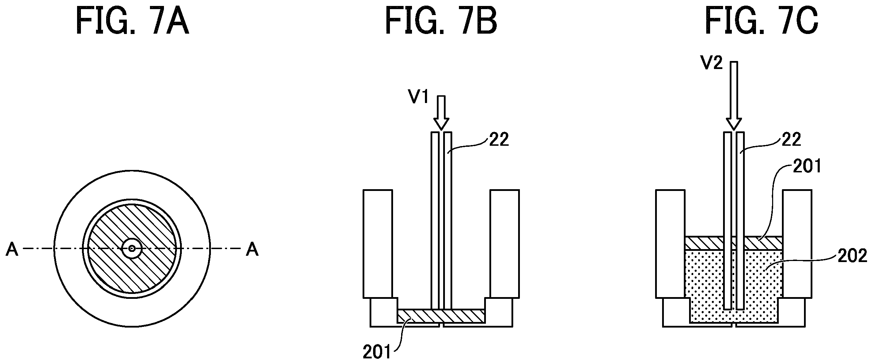

FIGS. 7A to 7C are schematic views of the liquid discharge unit 1 and the supply unit 2 during a supply process. FIG. 7A is a top view of the liquid discharge unit 1, and FIGS. 7B and 7C are cross-sectional views taken along line A-A in FIG. 7A.

FIG. 7B illustrates the supply process of the first liquid 201. In the supply process of the first liquid 201, the supply unit 2 supplies the first liquid 201 from the first reservoir 81 to the liquid discharge unit 1 (i.e., a first supply process). The first liquid 201 has a lower surface tension than the second liquid 202. Since the first liquid 201 having a small surface tension is likely to wet and spread, the first liquid 201 widely spreads to the entire liquid discharge unit 1, thereby preventing bubbles from being generated and remaining in the liquid discharge unit 1.

In the first supply process, the distance H from the lower surface 22a of the tube 22 to the upper surface 132b of the film 132 is preferably short when the first liquid 201 is fed to the liquid discharge unit 1. As a result, the kinetic energy of the first liquid 201 is small when the supplied first liquid 201 reaches the film 132, thereby preventing the first liquid 201 from vibrating and scattering. As a result, bubbles due to vibration or scattering of the first liquid 201 can be prevented from being generated.

FIG. 7C illustrates the supply process of the second liquid 202. In the supply process of the second liquid 202, the supply unit 2 supplies the second liquid 202 from the second reservoir 82 to the liquid discharge unit 1 (i.e., a second supply process). The second liquid 202 is supplied to the liquid discharge unit 1 to which the first liquid 201 has been supplied. Accordingly, the liquid discharge unit 1 can hold the second liquid 202 in a state in which bubbles are suppressed compared to when only the second liquid 202 is supplied to the liquid discharge unit 1.

In the second supply process, when the second liquid 202 is fed to the liquid discharge unit 1, the lower surface 22a of the tube 22 preferably contacts at least a part of the first liquid 201. For example, the lower surface 22a of the tube 22 is preferably in contact with the upper surface of the first liquid 201 or immersed in the first liquid 201. Thus, the kinetic energy of the second liquid 202 is small when the supplied second liquid 202 reaches the upper surface of the first liquid 201 previously supplied, thereby preventing the first liquid 201 and the second liquid 202 from vibrating and scattering. As a result, bubbles due to vibration or scattering of the first liquid 201 and the second liquid 202 can be suppressed.

As described above, in the present embodiment, the supply unit 2 supplies the second liquid 202 to the liquid discharge unit 1 after supplying the first liquid 201 having the small contact angle with the upper surface of the MEMS chip 13 to the liquid discharge unit 1. Therefore, the first liquid 201 having the small contact angle with the upper surface of the MEMS chip 13 reaches the corner or the upper surface of the film 132 in the liquid discharge unit 1 before the second liquid 202, thereby preventing bubbles from being generated and remaining in the liquid discharge unit 1.

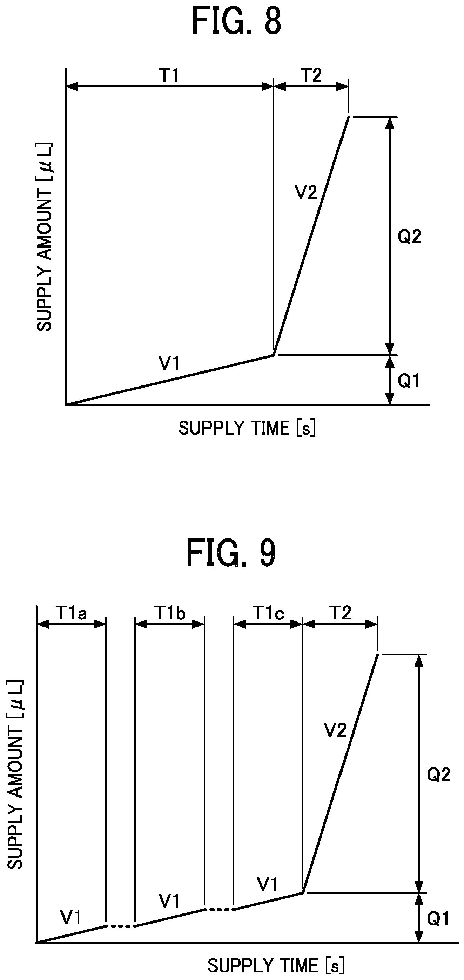

Next, the supply process is described in detail. As described above, the second supply process is performed after the first supply process to suppress the generation and remaining of bubbles, and it is more preferable to adopt a process illustrated in FIG. 8 or FIG. 9. Here, the first liquid 201 is supplied at a supply rate V1 in the first supply process, and the second liquid 202 is supplied at a supply rate V2 in the second supply process. Specifically, the supply rate V1 indicates that a predetermined supply amount Q1 of the first liquid 201 is supplied over a supply time T1, and the supply rate V2 indicates that a predetermined supply amount Q2 of the second liquid 202 is supplied over a supply time T2.

FIG. 8 is a graph illustrating an example of the supply process in which the first liquid 201 is supplied at a lower supply rate than the second liquid 202. As illustrated in FIG. 8, the supply rate V1 of the first liquid 201 in the first supply process is preferably lower than the supply rate V2 of the second liquid 202 in the second supply process (i.e., V1 < V2). The supply rate V1 is set to be lower than the supply rate V2, thereby further suppressing the generation of bubbles when the first liquid 201 is supplied. When the supply amounts Q1 and Q2 are changed, the supply times T1 and T2 are also changed accordingly, but the relation between the supply rates V1 and V2 (i.e., V1 < V2) is not changed.

FIG. 9 is a graph illustrating an example of the supply process in which the first liquid 201 is supplied intermittently. As illustrated in FIG. 9, the supply time T1 for supplying the supply amount Q1 of the first liquid 201 in the first supply process is preferably divided into supply times T1a, T1b, and T1c. In other words, the supply amount Q1 of the first liquid 201 in the first supply process is preferably divided into multiple supply amounts, and the multiple supply amounts are intermittently supplied little by little in multiple batches. Such an intermittent supply of the first liquid 201 can further suppress the generation of bubbles in the first supply process.

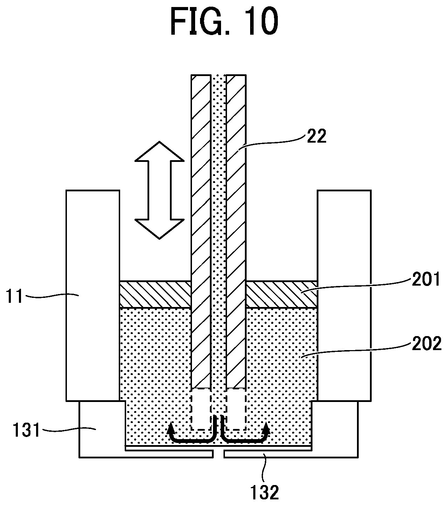

FIG. 10 is a schematic view of the supply unit 2 that stirs the second liquid 202 in the liquid discharge unit 1. To prevent precipitation of some ingredients of the second liquid 202, the second liquid 202 supplied to the liquid discharge unit 1 is preferably stirred in a stirring process. The supply unit 2 feeds and sucks the second liquid 202 to stir the second liquid (i.e., the stirring process).

The stirring process may be performed after the second liquid 202 has been supplied or may be performed while the second liquid 202 is supplied. When the stirring process is performed after the second liquid 202 has been supplied, the stirring process is preferably performed while keeping the tube 22, which is inserted into the liquid discharge unit 1, immersed in the second liquid 202. Thus, the second liquid 202 can be stirred while suppressing the generation of new bubbles in the first liquid 201 and the second liquid 202 supplied. Further, the tube 22 may be moved up and down while stirring the second liquid 202. Thus, the second liquid 202 can be uniformly stirred.

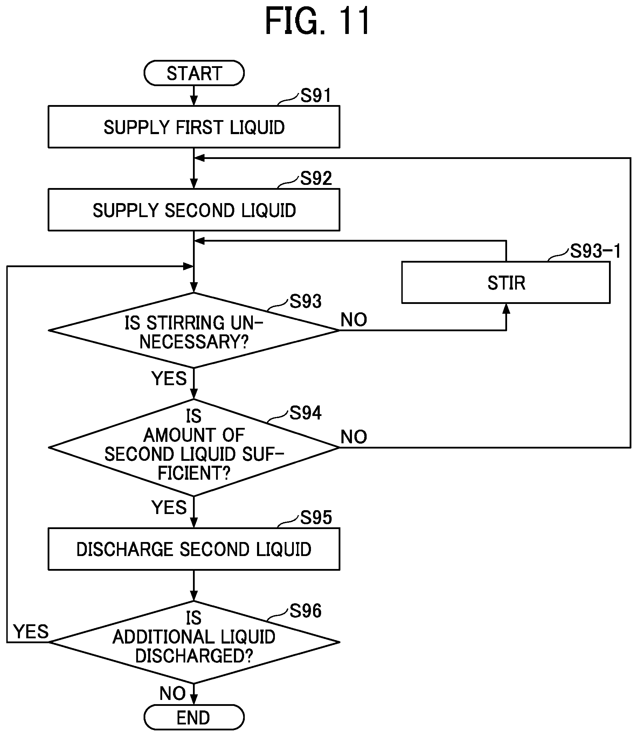

Next, a liquid discharging method (liquid filling method) using the liquid discharge apparatus 100 is described with reference to FIG. 11. FIG. 11 is a flowchart illustrating an example of the operation of the liquid discharge apparatus 100.

First, in step S91, the supply control unit 42 drives the supply unit 2 to supply the first liquid 201 to the liquid discharge unit 1 as the first supply process. Subsequently, in step S92, the supply control unit 42 drives the supply unit 2 to supply the second liquid 202 to the liquid discharge unit 1 as the second supply process.

Thereafter, in step S93, the stir control unit 43 determines whether or not to stir the second liquid 202. When the stir control unit 43 determines that it is necessary to stir the second liquid 202 (No in step S93), the stir control unit 43 causes the supply unit 2 to stir the second liquid 202 in step S93-1. When the stir control unit 43 determines that it is unnecessary to stir the second liquid 202 (Yes in step S93), the supply control unit 42 determines whether the amount of the second liquid 202 is sufficient in step S94.

When the supply control unit 42 determines that the amount of the second liquid 202 is insufficient (No in step S94), the process returns to step S92, and the operation of step S92 is performed again. When the supply control unit 42 determines that the amount of the second liquid 202 is sufficient (Yes in step S94), the discharge control unit 41 cause the liquid discharge unit 1 to discharge the second liquid 202 as the discharge process in step S95.

Subsequently, the supply control unit 42 determines whether or not to additionally discharge the second liquid 202 in step S96. When the supply control unit 42 determines that additional discharge of the second liquid 202 is necessary (Yes in step S96), the process returns to step S93, and the operation of step S93 is performed again. When the supply control unit 42 determines that additional discharge of the second liquid 202 is unnecessary (No in step S96), the liquid discharge apparatus 100 ends the operation.

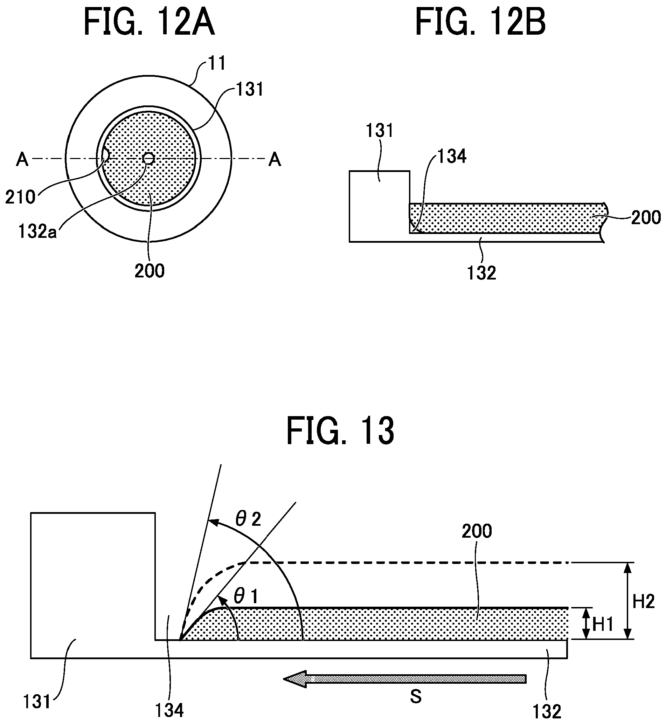

FIGS. 12A and 12B are schematic views of the liquid discharge unit 1 in which a bubble 210 remains. FIG. 12A is a top view of the liquid discharge unit 1, and FIG. 12B is a cross-sectional view of the liquid discharge unit 1 taken along line A-A in FIG. 12A. In FIGS. 12A and 12B, the bubble 210 remains in the corner 134 of the liquid discharge unit 1. As illustrated in FIG. 12B, when the liquid 200 does not reach the corner 134, where the inner surface of the film 132 and the inner surface of the holder 131 intersect each other, due to the surface tension of the liquid 200, a space is generated in the corner 134. Air in the space adheres to the wall surface as the bubble 210, and the bubble 210 is likely to remain in the corner 134. The bubble 210 may adhere to and remain on the upper surface of the film 132.

If bubbles remain in the liquid discharge unit 1, the bubbles absorb the pressure applied to the liquid 200 when the liquid 200 is discharged, and thus the liquid 200 may be discharged unstably. That is, the liquid discharge unit 1 may unstably discharge the liquid 200 when the liquid 200 contains the bubbles.

In the present embodiment, the first liquid 201 is supplied before the second liquid 202, and the contact angle of the first liquid 201 with the upper surface of the MEMS chip 13 is less than 90 degrees. Since the first liquid 201 having the small contact angle is likely to wet and spread, the first liquid 201 widely spreads to the entire liquid discharge unit 1 and reaches the corner 134. Thus, the generation and remaining of bubbles in the corner 134 can be suppressed. The second liquid 202 is supplied after the first liquid 201 reaches the corner 134. Therefore, the liquid discharge unit 1 can hold the second liquid 202 while suppressing bubbles.

FIG. 13 is a schematic view of the liquid discharge unit 1 illustrating a relation between a height of the liquid 200 and a contact angle. Specifically, FIG. 13 illustrates a state in which the liquid 200 wets and spreads on the upper surface of the film 132 in the direction indicated by arrow S when the liquid 200 is supplied to the liquid discharge unit 1. When a small amount of the liquid 200 is supplied at once, the liquid 200 having a height H1 wets and spreads on the upper surface of the film 132. When a large amount of the liquid 200, which is greater than the small amount of the liquid 200 having the height H1, is supplied at once, the liquid 200 having a height H2 wets and spreads on the upper surface of the film 132. The liquid 200 having the height H1 contacts the film 132 with a contact angle θ1, and the liquid 200 having the height H2 contacts the film 132 with a contact angle θ2. The liquid supply rate is the same when the liquid 200 has the height H1 and when the liquid 200 has the height H2.

As illustrated in FIG. 13, the contact angle θ1 of the liquid 200 having the height H1 with the film 132 is smaller than the contact angle θ2 of the liquid 200 having the height H2 with the film 132. That is, as the amount of the liquid 200 supplied at a time is smaller, the height of the liquid 200 becomes lower and the contact angle formed by the surface of the liquid 200 with the film 132 becomes smaller. Accordingly, the liquid 200 spreads widely and reaches the corner 134, thereby suppressing the generation of bubbles in the corner 134. The generation of bubbles on the upper surface of the film 132 is also suppressed. Further, when the supply amount of the liquid 200 is the same, the height of the liquid 200 becomes smaller at the lower supply rate. Thus, the low supply rate of the liquid 200 can suppress the generation of bubbles in the corner 134 and on the upper surface of the film 132.

Next, a liquid discharge apparatus 100a according to a second embodiment of the present disclosure is described. The same components described in the first embodiment are denoted by the same reference numerals, and redundant description thereof is omitted as appropriate. The same applies to other embodiments described below. The second embodiment is different from the first embodiment in that a drain process is provided after the second supply process.

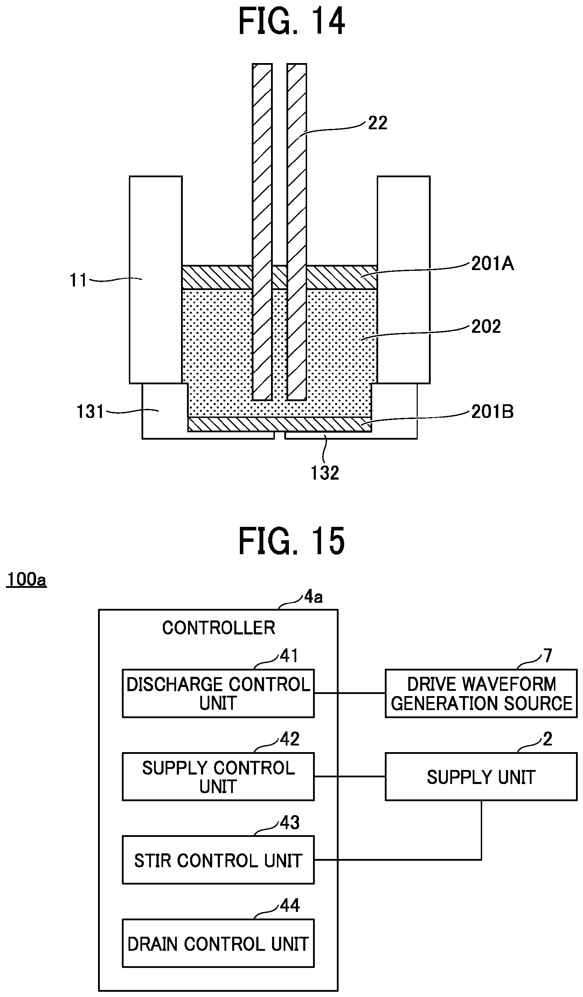

In the case where the first liquid 201 has the lower density than the second liquid 202, when the second liquid 202 is supplied in the second supply process, as described above, the first liquid 201 floats up to the upper surface of the second liquid 202 and covers the second liquid 202 like a lid. At that time, not all of the first liquid 201 floats up to the upper surface, and as illustrated in FIG. 14, the first liquid 201 may be separated into two portions and one of the separated first liquid 201 may remain at the bottom of the liquid discharge unit 1. Here, the bottom of the liquid discharge unit 1 refers to the vicinity of the film 132.

In FIG. 14, when the second liquid 202 is supplied, a portion 201A of the first liquid 201 floats up to the upper surface of the second liquid 202, and the other portion 201B of the first liquid 201 remains at the bottom of the liquid discharge unit 1. The other portion 201B of the first liquid 201 remaining at the bottom of the liquid discharge unit 1 is preferably drained to the outside of the liquid discharge unit 1 before the second liquid 202 is discharged to the well plate 400.

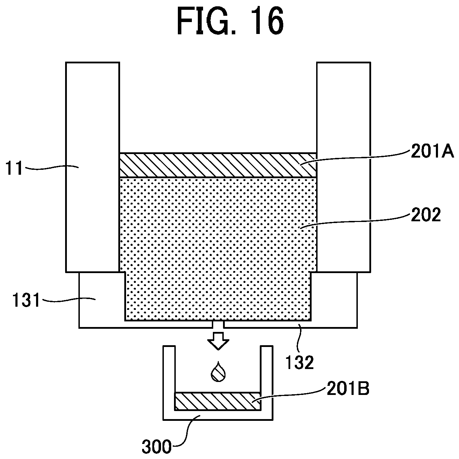

FIG. 15 is a block diagram illustrating an example of a functional configuration of a controller 4a included in the liquid discharge apparatus 100a. As illustrated in FIG. 15, the controller 4a includes a drain control unit 44. The drain control unit 44 controls drain of the first liquid 201. The drain control unit 44 may cause the drive waveform generation source 7 to apply a drive waveform to the MEMS chip 13 or may cause the supply unit 2 to suck the first liquid 201, to drain the other portion 201B of the first liquid 201. FIG. 16 is a schematic view of the liquid discharge unit 1 from which the first liquid 201 is drained. The other portion 201B of the first liquid 201 may be drained to a dedicated container 300 as illustrated in FIG. 16 or may be sucked by the supply unit 2.

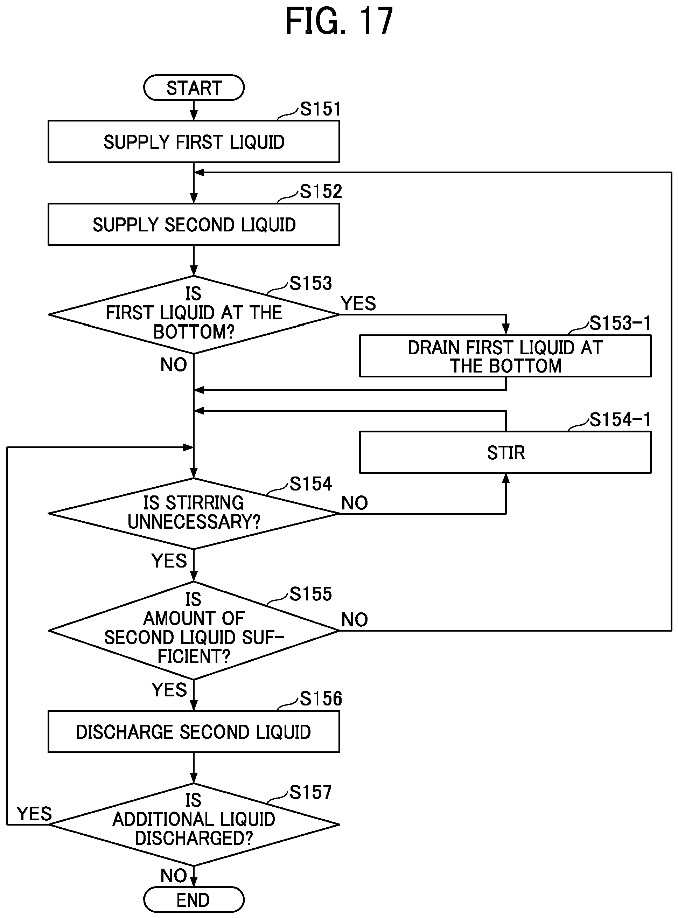

Next, a liquid discharge method (liquid filling method) using the liquid discharge apparatus 100a is described with reference to FIG. 17. FIG. 17 is a flowchart illustrating an example of the operation of the liquid discharge apparatus 100a.

First, in step S151, the supply control unit 42 drives the supply unit 2 to supply the first liquid 201 to the liquid discharge unit 1 as the first supply process. At that time, as described with reference to FIG. 8 or 9, the first liquid 201 may be supplied at a low supply rate or may be supplied intermittently in multiple batches. Subsequently, in step S152, the supply control unit 42 drives the supply unit 2 to supply the second liquid 202 to the liquid discharge unit 1 as the second supply process.

Thereafter, in step S153, the drain control unit 44 determines whether or not the first liquid is present at the bottom of the liquid discharge unit 1. When the drain control unit 44 determines that the first liquid 201 is present at the bottom of the liquid discharge unit 1 (Yes in step S153), the drain control unit 44 cause liquid discharge unit 1 to drain the first liquid 201 at the bottom in step S153-1. When the drain control unit 44 determines that the first liquid 201 is not present at the bottom of the liquid discharge unit 1 (No in step S153), the stir control unit 43 determines whether or not to stir the second liquid 202 in step S154.

When the stir control unit 43 determines that it is necessary to stir the second liquid 202 (No in step S154), the stir control unit 43 causes the supply unit 2 to stir the second liquid 202 in step S154-1. When the stir control unit 43 determines that it is unnecessary to stir the second liquid 202 (Yes in step S154), the supply control unit 42 determines whether the amount of the second liquid 202 is sufficient in step S155.

When the supply control unit 42 determines that the amount of the second liquid 202 is insufficient (No in step S155), the process returns to step S152, and the operation of step S152 is performed again. When the supply control unit 42 determines that the amount of the second liquid 202 is sufficient (Yes in step S155), the discharge control unit 41 cause the liquid discharge unit 1 to discharge the second liquid 202 as the discharge process in step S156.

Subsequently, the supply control unit 42 determines whether or not to additionally discharge the second liquid 202 in step S157. When the supply control unit 42 determines that additional discharge of the second liquid 202 is necessary (Yes in step S157), the process returns to step S154, and the operation of step S154 is performed again. When the supply control unit 42 determines that additional discharge of the second liquid 202 is unnecessary (No in step S157), the liquid discharge apparatus 100 ends the operation.

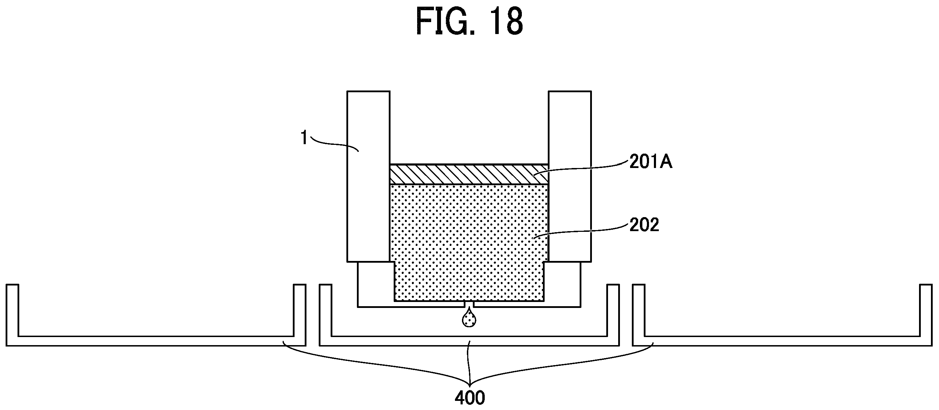

With the above configuration, as illustrated in FIG. 18, when the second liquid 202 is discharged to the well plate 400 as liquid droplets D, the first liquid 201 can be prevented from being contained in the liquid droplets D. That is, when the second liquid 202 is discharged to the well plate 400 as liquid droplets D, the first liquid 201 is not contained in the liquid droplets D, and only the second liquid 202 can be reliably discharged.

Next, the liquid discharge apparatus according to a third embodiment of the present disclosure is described. The liquid discharge apparatus according to the present embodiment includes at least a liquid discharge unit 1b. For example, the configuration of the liquid discharge apparatus 100 illustrated in FIG. 1 can be applied to that of the liquid discharge apparatus according to the present embodiment. For example, the configuration of the liquid discharge unit 1 illustrated in FIG. 3 can be applied to the configuration of the liquid discharge unit 1b. The liquid discharge unit 1b has the contact angle of the upper surface 132b (an example of a first surface) of the film 132 with water of less than 90 degrees. Here, the upper surface 132b faces the liquid holding portion 14 in the liquid discharge unit 1b. In the present embodiment, each of the first liquid 201 and the second liquid 202 contains water. In the present embodiment, the liquid discharge apparatus includes a supply unit in addition to the liquid discharge unit 1b, for example. The configuration of the supply unit 2 illustrated in FIG. 2 can be applied to that of the supply unit according to the present embodiment.

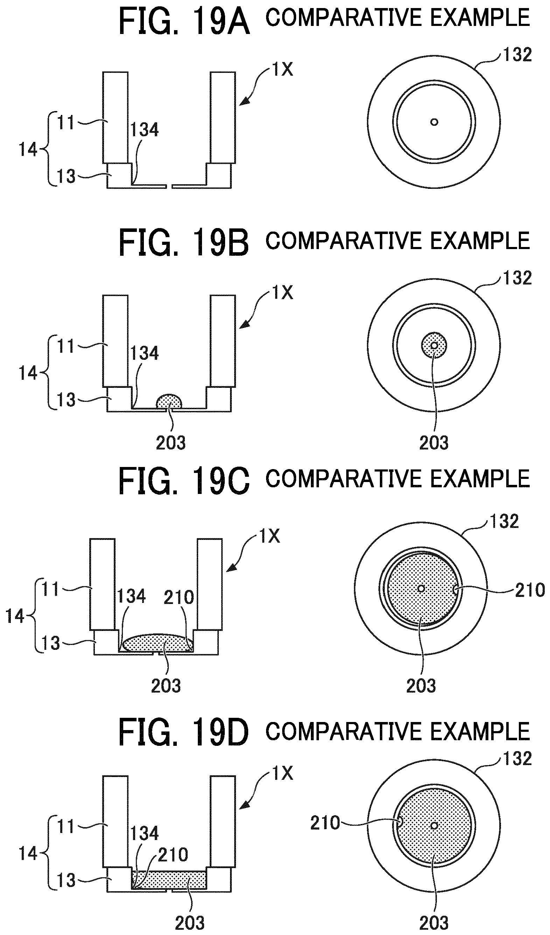

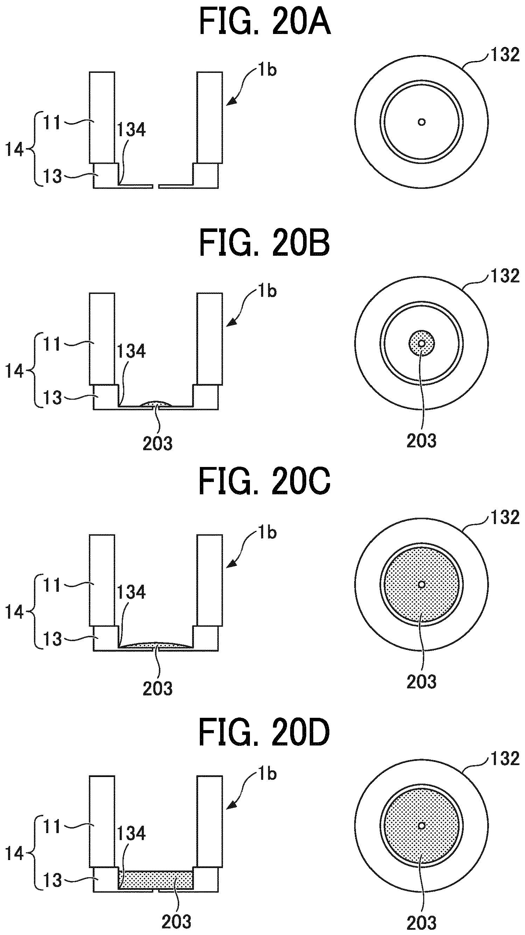

FIGS. 19A to 19D and FIGS. 20A to 20D are schematic views of the liquid discharge unit illustrating the operation of the liquid discharge apparatus. FIGS. 19A to 19D are schematic views of a liquid discharge unit 1X to which the present embodiment is not applied (i.e., a comparative example). In the liquid discharge unit 1X, the contact angle between the upper surface 132b of the film 132 and water is 90 degrees or more. FIGS. 20A to 20D are schematic views of the liquid discharge unit 1b according to the present embodiment. In the liquid discharge unit 1b, the contact angle between the upper surface 132b of the film 132 and water is less than 90 degrees. In FIGS. 19A to 19D and FIGS. 20A to 20D, only the contact angle of the upper surface 132b with water is different, and the same or similar components are denoted by the same reference numerals for convenience.

FIGS. 19A to 19D illustrate a process in which water 203 is supplied into the liquid holding portion 14 of the of the liquid discharge unit 1X in the order of FIGS. 19A, 19B, 19C, and 19D. Each of FIGS. 19A to 19D includes a cross-sectional view of the liquid discharge unit 1X on the left side and a top view of the film 132 on the right side. Similarly, FIGS. 20A to 20D illustrate a process in which water 203 is supplied into the liquid holding portion 14 of the liquid discharge unit 1b in the order of FIGS. 20A, 20B, 20C, and 20D. Each of FIGS. 20A to 20D includes a cross-sectional view of the liquid discharge unit 1b on the left side and a top view of the film 132 on the right side.

As illustrated in FIGS. 19A to 19D, the upper surface 132b of the film 132 has low wettability. Accordingly, water 203 supplied to the liquid holding portion 14 spreads on the upper surface 132b with the large contact angle. As a result, when the supply of the water 203 proceeds, the bubble 210 remains in the corner 134 of the liquid holding portion 14. As illustrated in FIGS. 20A to 20D, in the present embodiment, the upper surface 132b of the film 132 has good wettability to water 203 (i.e., the contact angle is less than 90 degrees). As a result, the water 203 supplied to the liquid holding portion 14 is likely to wet and spread on the film 132 of the liquid discharge unit 1b as compared with the liquid discharge unit 1X. When the supply of the water 203 proceeds, the bubble 210 can be prevented from remaining in the corner 134 of the liquid holding portion 14.

As a method for measuring the contact angle, for example, a sessile drop method in Japanese Industrial Standards (JIS) R 3257 "Test method for wettability of substrate glass surface" can be applied. The sessile drop method can be applied even to an object to be measured which does not contain a glass material.

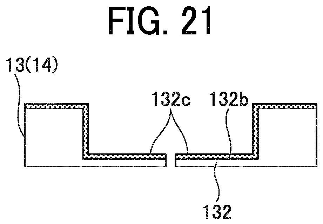

According to the invention, a film having hydrophilicity (hydrophilic film) is formed on the upper surface 132b to increase the wettability of the upper surface 132b so that the contact angle between the upper surface 132b and water is less than 90 degrees. The hydrophilic film can be formed by coating the upper surface 132b with a hydrophilic material or by modifying the upper surface 132b with plasma. The material having hydrophilicity is, for example, an oxide. In other words, the hydrophilic film includes an oxide, for example. FIG. 21 illustrates a hydrophilic film 132c formed on the upper surface 132b of the film 132. In the present embodiment, each of the first liquid 201 and the second liquid 202 contains water. Therefore, when the first liquid 201 or the second liquid 202 is supplied to the liquid holding portion 14, the same effect when water is supplied can be attained.

When a cell suspension is used as the second liquid 202, the concentration may become nonuniform due to sedimentation of cells or the nozzle hole 132a may be blocked due to sedimentation of cells. Therefore, preferably, the first liquid 201 or the second liquid 202 held in the liquid holding portion 14 is periodically stirred. In this case, if the surface roughness of the upper surface 132b of the film 132 is large, the sedimental cells may enter the unevenness of the upper surface 132b having the large surface roughness, and the stirring performance may be lowered. If a strong flow is generated to sufficiently stir the second liquid 202, the cells may be damaged. Therefore, the upper surface 132b preferably has high wettability and is smooth. The surface roughness of the upper surface 132b is preferably smaller than the size of the cells. For example, the surface roughness of the upper surface 132b preferably has a ten-point mean roughness Rz of 10 µm or less. The surface roughness can be measured by known techniques. The upper surface 132b can be coated to be smoothed. The liquid discharge apparatus having the smoothed upper surface 132b can make the concentration of the cell suspension uniform by stirring while preventing the cells from being damaged, and can prevent the nozzle hole 132a from being clogged.

As described in the first and second embodiments, not only the second liquid 202 but also the first liquid 201 may be supplied to the liquid holding portion 14 of the liquid discharge unit 1b. When a cell suspension is used as the second liquid 202, the usable solvent is limited in consideration of biocompatibility and subsequent culture. In addition, the surface treatment of the upper surface 132b of the film 132 may be limited due to restrictions on the performance and processing of the liquid discharge unit 1b. Due to this limitation, a desired contact angle may not be obtained depending on the combination of the upper surface 132b and the second liquid 202.

Therefore, in the variation of the third embodiment, the first liquid 201 has good wettability with respect to the liquid discharge unit 1b and is less likely to entrain the bubble 210. The second liquid 202 is supplied to the liquid holding portion 14 after the first liquid 201 is supplied to the liquid holding portion 14. Preferably, the first liquid 201 does not affect the second liquid 202 and the discharge of the second liquid 202. For example, when the second liquid 202 is a cell suspension, the first liquid 201 that is toxic to cells contained in the cell suspension is not used. Further, when the first liquid 201 and the second liquid 202 are mixed with each other, the concentration of the second liquid 202 may be lowered or the culture of cells may be affected. In addition, preferably, the first liquid 201 has a low specific gravity so as not to remaining in the vicinity of the nozzle hole 132a, and has a low surface tension so as not to entrain the bubble 210.

To satisfy the above conditions, the first liquid 201 is preferably silicone oil, liquid paraffin, or the like. The first liquid 201 preferably covers the entire surface of the second liquid 202, which is open to the atmosphere, like a lid to prevent the second liquid 202 from being exposed to the atmosphere. Thus, the second liquid 202 can be prevented from drying and volatilizing. As a result, a change in the concentration of the second liquid 202 can be suppressed.

In addition, the supply unit 2 preferably supplies the second liquid 202 to the liquid holding portion 14 after supplying the first liquid 201. Accordingly, the liquid discharge apparatus can spread the first liquid 201 over the entire liquid holding portion 14, and can reliably suppress the generation of the bubble 210 in the corner 134. The supply unit 2 can supply the first liquid 201 to the liquid holding portion 14 in multiple batches to more reliably suppress the generation of the bubble 210.

The surface tension of the first liquid 201 is preferably 25 mN/m or less. As a result, when the first liquid 201 is supplied to the liquid holding portion 14, the first liquid 201 can wet and spread, and the generation of the bubble 210 in the corner 134 can be suppressed. For example, an experiment was performed in which a liquid containing 70% ethanol and having a surface tension of 26 mN/m was supplied to the liquid holding portion 14. As a result, the bubble 210 is generated in the liquid holding portion 14. Therefore, the surface tension of the first liquid 201 is preferably 25 mN/m or less, which is smaller than 26 mN/m.

Preferably, the film 132 is disposed below the liquid holding portion 14 in the vertical direction, the first liquid 201 and the second liquid 202 are insoluble in each other, and the first liquid 201 is nonvolatile and have a specific gravity lower than that of the second liquid 202. Further, the first liquid 201 is preferably supplied so as to cover the entire upper surface of the second liquid 202. Accordingly, in the liquid discharge apparatus, the first liquid 201 can be disposed on the upper surface of the second liquid 202 supplied to the liquid holding portion 14, thereby preventing the second liquid 202 from contacting the atmosphere and drying due to the contact with the atmosphere.

After the first liquid 201 and the second liquid 202 are supplied to the liquid holding portion 14, the liquid discharge unit 1b preferably drains the first liquid 201 held on the bottom of the liquid holding portion 14, thereby removing the first liquid 201 from the inside of the liquid holding portion 14. Accordingly, when the second liquid 202 is supplied to the liquid holding portion 14, the liquid discharge apparatus can remove the first liquid 201 remaining on the film 132 in the liquid holding portion 14. As a result, the liquid discharge apparatus can discharge only the second liquid 202 that is originally intended to be discharged from the nozzle hole 132a of the film 132.

When the first liquid 201 is liquid to be discharged by the MEMS chip 13, preferably, the film 132 is disposed below the liquid holding portion 14 in the vertical direction, the first liquid 201 and the second liquid 202 are insoluble in each other, and the second liquid 202 is nonvolatile and have a specific gravity lower than that of the first liquid 201. Further, the second liquid 202 is preferably supplied so as to cover the entire upper surface of the first liquid 201. As a result, the liquid discharge apparatus can supply the second liquid 202 after the first liquid 201 to prevent the first liquid 201 from drying.

The supply rate of the first liquid 201 to the liquid holding portion 14 is preferably lower than the supply rate of the second liquid 202. Accordingly, the liquid discharge apparatus can improve the effect of preventing the generation of the bubble 210.

The second liquid 202 contains cells, and preferably, the first liquid 201 is not toxic to the cells contained in the second liquid 202. Thus, the liquid discharge apparatus can use a cell suspension as the second liquid 202.

The first liquid 201 is preferably one of silicone oil and liquid paraffin. This allows the first liquid 201 to satisfy the conditions of low specific gravity, low surface tension, and no toxicity to cells.

Although some embodiments have been described above, embodiments of the present disclosure are not limited to the above-described embodiments specifically disclosed, and various modifications and changes can be made without departing from the scope of the appended claims.

In addition, the numbers such as ordinal numbers and quantities used in the above-described embodiments are all examples for specifically describing the technology of the present disclosure, and embodiments of the present disclosure are not limited to the exemplified numbers. In addition, the above-describe connections among the components are examples for specifically describing the technology of the present disclosure, and connections for implementing functions of the present disclosure are not limited to the above-described examples.

Any one of the above-described operations may be performed in various other ways, for example, in an order different from the one described above.

The present invention can be implemented in any convenient form, for example using dedicated hardware, or a mixture of dedicated hardware and software. The present invention may be implemented as computer software implemented by one or more networked processing apparatuses. The processing apparatuses include any suitably programmed apparatuses such as a general purpose computer, a personal digital assistant, a Wireless Application Protocol (WAP) or third-generation (3G)-compliant mobile telephone, and so on. Since the present invention can be implemented as software, each and every aspect of the present invention thus encompasses computer software implementable on a programmable device. The computer software can be provided to the programmable device using any conventional carrier medium (carrier means). The carrier medium includes a transient carrier medium such as an electrical, optical, microwave, acoustic or radio frequency signal carrying the computer code. An example of such a transient medium is a Transmission Control Protocol/Internet Protocol (TCP/IP) signal carrying computer code over an IP network, such as the Internet. The carrier medium may also include a storage medium for storing processor readable code such as a floppy disk, a hard disk, a compact disc read-only memory (CD-ROM), a magnetic tape device, or a solid state memory device.

## Claims

1. A liquid discharge apparatus (100) comprising:
a liquid discharge unit (1) including:
a liquid chamber member (11) defining a liquid holding portion (14) to hold a liquid;
a film (132) having a nozzle hole (132a) from which the liquid is discharged and a surface (132b) facing the liquid holding portion (14), the surface (132b) having a contact angle with water of less than 90 degrees; and
a holder (131) supporting the film (132),
wherein the surface (132b) includes a hydrophilic film (132c).

2. The liquid discharge apparatus (100) according to claim 1,
wherein the hydrophilic film (132c) includes an oxide.

3. The liquid discharge apparatus (100) according to any one of claims 1 to 2, wherein the surface (132b) has a ten-point mean roughness of 10 µm or less.

4. The liquid discharge apparatus (100) according to any one of claims 1 to 3, further comprising a supply unit (2) configured to supply the liquid to the liquid holding portion (14) in multiple batches or in a single batch.

5. The liquid discharge apparatus (100) according to claim **4,**
wherein the supply unit (2) is configured to feed the liquid to and suck the liquid from the liquid holding portion (14) to stir the liquid.

6. A liquid filling method comprising:
supplying (S91; S151) a liquid to a liquid holding portion (14) of a liquid discharge unit (1),
wherein the liquid discharge unit (1) includes a film (132) having a nozzle hole (132a) from which the liquid is discharged and a surface (132b) facing the liquid holding portion (14), and the surface (132b) has a contact angle with the liquid of less than 90 degrees,
wherein the surface (132b) includes a hydrophilic film (132c).

7. The liquid filling method according to claim 6,
wherein the liquid has a surface tension of 25 mN/m or less.

8. The liquid filling method according to claim 6 or 7, further comprising supplying (S92; S152) another liquid to the liquid holding portion (14) after the supplying (S91; S151) of the liquid to the liquid holding portion (14).

9. The liquid filling method according to claim 8,
wherein the liquid is insoluble in said another liquid, is nonvolatile, and has a lower specific gravity than said another liquid, and
wherein the liquid supplied to the liquid holding portion (14) covers an entire upper surface of said another liquid.

10. The liquid filling method according to claim 9, further comprising draining (S153-1) the liquid on the surface of the film (132) to remove the liquid after the supplying (S92; S152) of said another liquid to the liquid holding portion (14).

11. The liquid filling method according to claim 8,
wherein the liquid is insoluble in said another liquid,
wherein said another liquid is nonvolatile and has a lower specific gravity than the liquid, and
wherein said another liquid supplied to the liquid holding portion (14) covers an entire upper surface of the liquid.

12. The liquid filling method according to any one of claims 8 to 11,
wherein a rate of the supplying (S91; S151) of the liquid to the liquid holding portion (14) is lower than a rate of the supplying (S92; S152) of said another liquid to the liquid holding portion (14).

13. The liquid filling method according to any one of claims 8 to 12,
wherein one of the liquid and said another liquid includes cells, and another of the liquid and said another liquid is a non-cytotoxic liquid that is not toxic to the cells.

14. The liquid filling method according to claim 13,
wherein the non-cytotoxic liquid comprises one of silicone oil and liquid paraffin.

## Patentansprüche

1. Flüssigkeitsabgabeeinrichtung (100), umfassend:
eine Flüssigkeitsabgabeeinheit (1), welche Folgendes beinhaltet:
ein Flüssigkeitskammerelement (11), das einen Flüssigkeitshalteabschnitt (14) definiert, um eine Flüssigkeit zu halten;
einen Film (132), der ein Düsenloch (132a), aus dem die Flüssigkeit abgegeben wird, und eine Oberfläche (132b) aufweist, die dem Flüssigkeitshalteabschnitt (14) zugewandt ist, wobei die Oberfläche (132b) einen Kontaktwinkel mit Wasser von weniger als 90 Grad aufweist; und
einen Halter (131), der den Film (132) trägt,
wobei die Oberfläche (132b) einen hydrophilen Film (132c) beinhaltet.

2. Flüssigkeitsabgabeeinrichtung (100) nach Anspruch 1,
wobei der hydrophile Film (132c) ein Oxid beinhaltet.

3. Flüssigkeitsabgabeeinrichtung (100) nach einem der Ansprüche 1 bis 2,
wobei die Oberfläche (132b) eine mittlere Rauheit an zehn Punkten von 10 µm oder weniger aufweist.

4. Flüssigkeitsabgabeeinrichtung (100) nach einem der Ansprüche 1 bis 3, die weiter eine Zufuhreinheit (2) umfasst, die so ausgelegt ist, dass sie dem Flüssigkeitshalteabschnitt (14) die Flüssigkeit in mehreren Chargen oder in einer einzigen Charge zuführt.

5. Flüssigkeitsabgabeeinrichtung (100) nach Anspruch 4,
wobei die Zufuhreinheit (2) so ausgelegt ist, dass sie die Flüssigkeit zu dem Flüssigkeitshalteabschnitt (14) leitet und die Flüssigkeit aus diesem saugt, um die Flüssigkeit zu rühren.

6. Flüssigkeitsfüllverfahren, umfassend:
Zuführen (S91; S151) einer Flüssigkeit zu einem Flüssigkeitshalteabschnitt (14) einer Flüssigkeitsabgabeeinheit (1),
wobei die Flüssigkeitsabgabeeinheit (1) einen Film (132) beinhaltet, der ein Düsenloch (132a), aus dem die Flüssigkeit abgegeben wird, und eine Oberfläche (132b) aufweist, die dem Flüssigkeitshalteabschnitt (14) zugewandt ist, und die Oberfläche (132b) einen Kontaktwinkel mit der Flüssigkeit von weniger als 90 Grad aufweist,
wobei die Oberfläche (132b) einen hydrophilen Film (132c) beinhaltet.

7. Flüssigkeitsfüllverfahren nach Anspruch 6,
wobei die Flüssigkeit eine Oberflächenspannung von 25 mN/m oder weniger aufweist.

8. Flüssigkeitsfüllverfahren nach Anspruch 6 oder 7, das weiter das Zuführen (S92; S152) einer anderen Flüssigkeit zum Flüssigkeitshalteabschnitt (14) nach dem Zuführen (S91; S151) der Flüssigkeit zum Flüssigkeitshalteabschnitt (14) umfasst.

9. Flüssigkeitsfüllverfahren nach Anspruch 8,
wobei die Flüssigkeit in der anderen Flüssigkeit unlöslich ist, nicht flüchtig ist und ein geringeres spezifisches Gewicht als die andere Flüssigkeit aufweist, und
wobei die dem Flüssigkeitshalteabschnitt (14) zugeführte Flüssigkeit eine gesamte obere Oberfläche der anderen Flüssigkeit bedeckt.

10. Flüssigkeitsfüllverfahren nach Anspruch 9, das weiter das Ablassen (S153-1) der Flüssigkeit auf der Oberfläche des Films (132) umfasst, um die Flüssigkeit nach dem Zuführen (S92; S152) der anderen Flüssigkeit zum Flüssigkeitshalteabschnitt (14) zu entfernen.

11. Flüssigkeitsfüllverfahren nach Anspruch 8,
wobei die Flüssigkeit in der anderen Flüssigkeit unlöslich ist,
wobei die andere Flüssigkeit nichtflüchtig ist und ein geringeres spezifisches Gewicht als die Flüssigkeit aufweist, und
wobei die dem Flüssigkeitshalteabschnitt (14) zugeführte andere Flüssigkeit eine gesamte obere Oberfläche der Flüssigkeit bedeckt.

12. Flüssigkeitsfüllverfahren nach einem der Ansprüche 8 bis 11,
wobei eine Rate des Zuführens (S91; S151) der Flüssigkeit zum Flüssigkeitshalteabschnitt (14) geringer ist als eine Rate des Zuführens (S92; S152) der anderen Flüssigkeit zum Flüssigkeitshalteabschnitt (14).

13. Flüssigkeitsfüllverfahren nach einem der Ansprüche 8 bis 12,
wobei eine der Flüssigkeit und der anderen Flüssigkeit Zellen beinhaltet und die andere der Flüssigkeit und der anderen Flüssigkeit eine nicht-zytotoxische Flüssigkeit ist, die für die Zellen nicht toxisch ist.

14. Flüssigkeitsfüllverfahren nach Anspruch 13,
wobei die nicht-zytotoxische Flüssigkeit eines von Silikonöl und flüssigem Paraffin umfasst.

## Revendications

1. Appareil d'évacuation de liquide (100) comprenant :
une unité d'évacuation de liquide (1) incluant :
un organe chambre à liquide (11) définissant une partie contenant du liquide (14) pour contenir un liquide ;
un film (132) présentant un trou de buse (132a) duquel le liquide est évacué et une surface (132b) faisant face à la partie contenant du liquide (14), la surface (132b) présentant un angle de contact avec l'eau inférieur à 90 degrés ; et
un support (131) supportant le film (132),
dans lequel la surface (132b) inclut un film hydrophile (132c).

2. Appareil d'évacuation de liquide (100) selon la revendication 1,
dans lequel le film hydrophile (132c) inclut un oxyde.

3. Appareil d'évacuation de liquide (100) selon l'une quelconque des revendications 1 à 2,
dans lequel la surface (132b) présente une rugosité moyenne en dix points de 10 µm ou moins.

4. Appareil d'évacuation de liquide (100) selon l'une quelconque des revendications 1 à 3, comprenant en outre une unité d'alimentation (2) configurée pour alimenter en liquide la partie contenant du liquide (14) en plusieurs fois ou en une seule fois.

5. Appareil d'évacuation de liquide (100) selon la revendication 4,
dans lequel l'unité d'alimentation (2) est configurée pour introduire le liquide dans et aspirer le liquide de la partie contenant du liquide (14) pour remuer le liquide.

6. Procédé de remplissage de liquide comprenant :
l'alimentation (S91 ; S151) en liquide d'une partie contenant du liquide (14) d'une unité d'évacuation de liquide (1),
dans lequel l'unité d'évacuation de liquide (1) inclut un film (132) présentant un trou de buse (132a) duquel le liquide est évacué et une surface (132b) faisant face à la partie contenant du liquide (14), et la surface (132b) présente un angle de contact avec le liquide inférieur à 90 degrés,
dans lequel la surface (132b) inclut un film hydrophile (132c).

7. Procédé de remplissage de liquide selon la revendication 6,
dans lequel le liquide présente une tension superficielle de 25 mN/m ou moins.

8. Procédé de remplissage de liquide selon la revendication 6 ou 7, comprenant en outre l'alimentation (S92 ; S152) en un autre liquide de la partie contenant du liquide (14) après l'alimentation (S91; S151) en liquide de la partie contenant du liquide (14).

9. Procédé de remplissage de liquide selon la revendication 8,
dans lequel le liquide est insoluble dans ledit autre liquide, est non volatil et présente une densité inférieure à celle dudit autre liquide, et
dans lequel le liquide fourni à la partie contenant du liquide (14) recouvre toute une surface supérieure dudit autre liquide.

10. Procédé de remplissage de liquide selon la revendication 9, comprenant en outre la vidange (S153-1) du liquide sur la surface du film (132) pour éliminer le liquide après l'alimentation (S92 ; S152) en ledit autre liquide de la partie contenant du liquide (14).

11. Procédé de remplissage de liquide selon la revendication 8,
dans lequel le liquide est insoluble dans ledit autre liquide,
dans lequel ledit autre liquide est non volatil et présente une densité inférieure à celle du liquide, et
dans lequel ledit autre liquide fourni à la partie contenant du liquide (14) recouvre toute une surface supérieure du liquide.

12. Procédé de remplissage de liquide selon l'une quelconque des revendications 8 à 11,
dans lequel un débit d'alimentation (S91 ; S151) en liquide vers la partie contenant du liquide (14) est inférieur à un débit d'alimentation (S92 ; S152) en ledit autre liquide vers la partie contenant du liquide (14).

13. Procédé de remplissage de liquide selon l'une quelconque des revendications 8 à 12,
dans lequel l'un du liquide et dudit autre liquide inclut des cellules, et l'autre du liquide et dudit autre liquide est un liquide non cytotoxique qui n'est pas toxique pour les cellules.

14. Procédé de remplissage de liquide selon la revendication 13,
dans lequel le liquide non cytotoxique inclut soit de l'huile de silicone, soit de la paraffine liquide.
